# EUROPEAN PATENT APPLICATION

(11) **EP 2 497 832 A1**
(43) Date of publication of application: **12.09.2012**
(21) Application number: 10828270.8
(22) Date of filing: 01.11.2010
(51) Int. Cl.: C12P 13/04, C12N 15/09, C12P 13/08

(54) **METHOD FOR PRODUCING L-AMINO ACID**

(30) Priority: 06.11.2009 JP 2009255042
(71) Applicant: Ajinomoto Co., Inc., Tokyo 104-8315 (JP)
(72) Inventor: INOUE, Ippei, Kawasaki-shi Kanagawa 210-8681 (JP); YASUEDA, Hisashi, Kawasaki-shi Kanagawa 210-8681 (JP)
(74) Representative: Strehl Schübel-Hopf & Partner
(86) International application number: PCT/JP2010/069436
(87) International publication number: WO 2011/055710

(57) **Abstract**

A method for efficiently producing an L-amino acid, especially L-lysine, by using a γ-proteobacterium is provided. In a method for producing an L-amino acid comprising culturing a bacterium belonging to γ-*Proteobacteria* and having an ability to produce an L-amino acid, for example, an *Enterobacteriaceae* bacterium such as *Escherichia coli,* in a medium containing glycerol as a carbon source to produce and accumulate the L-amino acid in the medium, and collecting the L-amino acid from the medium, a bacterium modified so that the activity of the Cnu protein is reduced is used as the bacterium.

## Description

### Technical Field

The present invention relates to a method for producing an L-amino acid utilizing a Υ-proteobacterium, and especially for producing L-lysine. L-Lysine is an industrially useful L-amino acid as an additive for animal feed, ingredient of health food, amino acid infusion solution, and so forth.

### Background Art

In *Escherichia coli,* the *ydgT* gene codes for a protein having a full length of 71 amino acid residues called Cnu (oriC-binding nucleoid-associated) protein (Non-patent document 1), and it has been thought that the Cnu protein belongs to the Hha/YmoA family of gene regulation factors that respond to environmental changes on the basis of the amino acid sequence thereof (Non-patent document 2). It has also been thought that the *ydgT* gene product of *Salmonella* bacteria regulates expression of the region SPI-2 coding for genes involved in systemic infection of *Salmonella* bacteria in animals through interactions with the H-NS protein (Non-patent document 3).

However, involvement of the *ydgT* gene in metabolism, such as saccharide consumption and amino acid production, has not been understood well for any bacteria.

### Prior art references

### Non-patent documents

Non-patent document 1: Kim, M.S. et al., J. Bacteriol., 187:6998-7008 (2005)
Non-patent document 2: Paytubi, S. et al., Mol. Microbiol., 54 (1) :251-63 (2004)
Non-patent document 3: Coombes, B.K. et al., Proc. Natl. Acad. Sci. U.S.A., 102:17460 (2005)

### Summary of the Invention

### Object to be Achieved by the Invention

An object of the present invention is to provide a method for efficiently producing an L-amino acid, especially L-lysine, by using a Υ-proteobacterium.

### Means for Achieving the Object

The inventors of the present invention conducted various researches in order to achieve the aforementioned object, as a result, found that L-amino acids could be efficiently produced by using a bacterium in which functional activity of the Cnu protein was reduced, and thus accomplished the present invention.

That is, the present invention is embodied as follows.
(1) A method for producing an L-amino acid comprising culturing a bacterium belonging to Υ*-Proteobacteria* and having an ability to produce the L-amino acid in a medium containing glycerol as a carbon source to produce and accumulate the L-amino acid in the medium, and collecting the L-amino acid from the medium, wherein the bacterium is a bacterium modified so that the activity of the Cnu protein is reduced.
(2) The method as mentioned above, wherein the activity of the Cnu protein is reduced by disrupting the *ydgT* gene coding for the Cnu protein on the chromosome, or by reducing expression amount of the gene.
(3) The method as mentioned above, wherein the Cnu protein is a protein defined in the following (A) or (B):
   (A) a protein having the amino acid sequence shown in SEQ ID NO: 14,
   (B) a protein having the amino acid sequence shown in SEQ ID NO: 14, but including substitution, deletion, insertion, or addition of one or several amino acid residues, reduction of which activity in the bacterium results in improvement in the ability to produce the L-amino acid.
(4) The method as mentioned above, wherein the *ydgT* gene is a DNA defined in the following (a) or (b):
   (a) a DNA comprising the nucleotide sequence of SEQ ID NO: 13,
   (b) a DNA hybridizable with a nucleotide sequence complementary to the nucleotide sequence of SEQ ID NO: 13 or a probe that can be prepared from the nucleotide sequence under stringent conditions, and coding for a protein, reduction of which activity in the bacterium results in improvement in the ability to produce the L-amino acid.
(5) The method as mentioned above, wherein the L-amino acid is an aspartic acid type amino acid.
(6) The method as mentioned above, wherein the L-amino acid is L-lysine.
(7) The method as mentioned above, wherein the bacterium is a bacterium belonging to the family *Enterobacteriaceae.*
(8) The method as mentioned above, wherein the bacterium is an *Escherichia* bacterium.
(9) The method as mentioned above, wherein the bacterium is *Escherichia coli.*

### Effect of the Invention

According to the method of the present invention, L-amino acids, especially L-lysine, can be extremely efficiently produced by fermentation. Moreover, according to one embodiment of the present invention, the proliferation rate and glycerol-assimilating ability of bacteria are improved.

### Brief Description of the Drawings

[Fig. 1] Fig. 1 includes graphs showing (a) L-lysine concentrations, and (b) glycerol concentrations in an L-lysine production medium observed when a control strain and a *ydgT* gene-deficient strain of *Escherichia coli* were cultured in the medium.
[Fig. 2] Fig. 2 includes graphs showing (a) growth curves, and (b) glycerol concentrations in an L-lysine production medium observed when a control strain and a *ydgT* gene-deficient strain of *Escherichia coli* were cultured in the medium.

### Modes for Carrying out the Invention

### <1> Υ-Proteobacterium of the present invention

The Υ-proteobacterium used in the present invention is not particularly limited, so long as it is a microorganism that belongs to Υ-Proteobacteria, and has an ability to produce an L-amino acid or can be imparted with the ability to produce L-lysine. The bacterium of the present invention can be obtained by modifying a bacterium belonging to Υ*-Proteobacteria* and having an ability to produce an L-amino acid so that the activity of the Cnu protein is reduced.

Bacteria used as a parent strain of the bacterium of the present invention, which is to be modified so that expression amount of the *ydgT* gene is reduced, and methods for imparting or enhancing an L-amino acid-producing ability are exemplified below. The bacterium of the present invention can also be obtained by imparting an L-amino acid-producing ability to a Υ-proteobacterium modified so that expression amount of the *ydgT* gene is reduced, or enhancing an L-amino acid-producing ability of a Υ-proteobacterium modified so that expression amount of the *ydgT* gene is reduced.

In the present invention, a bacterium having an L-amino acid-producing ability (a bacterium having an ability to produce an L-amino acid) refers to a bacterium that has an ability to produce and accumulate an L-amino acid in a medium when it is cultured in the medium. The bacterium having an L-amino acid-producing ability also refers to a bacterium having an ability to accumulate an objective L-amino acid in a medium in an amount of preferably 0.5 g/L or more, more preferably 1.0 g/L or more.

In the present invention, examples of the L-amino acid include L-lysine, L-glutamic acid, L-threonine, L-valine, L-leucine, L-isoleucine, L-serine, L-aspartic acid, L-asparagine, L-glutamine, L-arginine, L-cysteine (cystine), L-methionine, L-phenylalanine, L-tryptophan, L-tyrosine, L-glycine, L-alanine, L-proline, L-ornithine, L-citrulline, and L-homoserine, and an aspartic acid type amino acid or an aromatic amino acid is especially preferred. Examples of the aspartic acid type amino acid include L-lysine, L-threonine, and L-methionine, and L-lysine is especially preferred. Examples of the aromatic amino acid include L-tryptophan, L-phenylalanine, and L-tyrosine. The L-amino acid can consist of one kind of L-amino acid, or two or more kinds of L-amino acids.

In the present invention, the L-amino acid includes not only an L-amino acid in free form, but also a salt thereof, such as sulfate, hydrochloride, carbonate, ammonium salt, sodium salt, and potassium salt.

### <1-1> Bacteria to be used as parent strain of the bacterium of the present invention

The bacterium of present invention is a bacterium belonging to Υ*-Proteobacteria* and having an L-amino acid-producing ability.
*Υ-Proteobacteria* include bacteria belonging to the family *Enterobacteriaceae,* such as those of genera *Escherichia, Enterobacter, Erwinia, Klebsiella, Pantoea, Photorhabdus, Providencia, Salmonella, Serratia, Shigella, Morganella,* and *Yersinia,* and bacteria belonging to the genus *Vibrio* and so forth. In the present invention, in particular, bacteria classified into Υ*-Proteobacteria* according to the taxonomy used in the NCBI (National Center for Biotechnology Information) database (http://www.ncbi.nlm.nih.gov/Taxonomy/Browser/wwwtax.cgi?id =91347) can be mentioned, and it is preferable to use an *Escherichia* or *Pantoea* bacterium belonging to the family *Enterobacteriaceae,* or a *Vibrio* bacterium.

A bacterium belonging to the genus *Escherichia* means that the bacterium is classified into the genus *Escherichia* according to classification known to a person skilled in the art of microbiology, although the bacterium is not particularly limited. Examples of the bacterium belonging to the genus *Escherichia* used in the present invention include, but are not limited to, *Escherichia coli (E. coli).*

The bacterium belonging to the genus *Escherichia* that can be used in the present invention is not particularly limited. However, examples include, for example, the bacteria of the phyletic groups described in the work of Neidhardt et al. (Neidhardt F.C. Ed., 1996, Escherichia coli and Salmonella: Cellular and Molecular Biology/Second Edition, pp.2477-2483, Table 1, American Society for Microbiology Press, Washington, D.C.). Specific examples include the *Escherichia coli* W3110 (ATCC 27325), *Escherichia coli* MG1655 (ATCC 47076), and the like derived from the prototype wild-type strain, K12 strain.

These strains are available from, for example, the American Type Culture Collection (Address: P.O. Box 1549, Manassas, VA 20108, United States of America). That is, registration numbers are given to each of the strains, and the strains can be ordered by using these numbers. The registration numbers of the strains are listed in the catalogue of the American Type Culture Collection.

A bacterium belonging to the genus *Pantoea* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii,* or the like, on the basis of the nucleotide sequence analysis of 16S rRNA, etc. (Int. J. Syst. Bacteriol., 43, 162-173 (1993)). In the present invention, bacteria belonging to the genus *Pantoea* encompass such bacteria re-classified into the genus *Pantoea* as described above.

The *Vibrio* bacteria are facultative anaerobic gram-negative bacteria belonging to the family *Vibrionanceae* of *Υ-Proteobacteria,* and are motile bacteria with one localized flagellum seen in fresh water or seawater. The *Vibrio* bacteria used in the present invention are desirably nonpathogenic *Vibrio* bacteria. *Vibrio* bacteria for which pathogenicity is not known are mentioned in Biosafety level 1 (Biosafety in Microbiological and Biomedical Laboratories (BMBL), 4th Edition, published by Office of Health and Safety (OHS)), and such *Vibrio* bacteria as mentioned below can be used.

*Vibrio abalonicus* ATCC 27390
*Vibrio adaptatus* ATCC 19263
*Vibrio aerogenes* ATCC 700797
*Vibrio aestuarianus* ATCC 35048
*Vibrio alginolyticus* ATCC 14582
*Vibrio algosus* ATCC 14390
*Vibrio anguillarum* ATCC 43305
*Vibrio calviensis* ATCC BAA-606
*Vibrio campbellii* ATCC 25920
*Vibrio carchariae* ATCC 35084
*Vibrio coralliilyticus* ATCC BAA-450
*Vibrio costicola* ATCC 43147
*Vibrio cyclitrophicus* ATCC 700982
*Vibrio cyclosites* ATCC 14635
*Vibrio diazotrophicus* ATCC 33466
*Vibrio fischeri* ATCC 25918
*Vibrio gazogenes* ATCC 29988
*Vibrio halioticoli* ATCC 700680
*Vibrio harveyi* ATCC 14126
*Vibrio hispanica* ATCC 51589
*Vibrio ichthyoenteri* ATCC 700023
*Vibrio iliopiscarius* ATCC 51760
*Vibrio lentus* ATCC BAA-539
*Vibrio liquefaciens* ATCC 17058
*Vibrio logei* ATCC 15382
*Vibrio marinagilis* ATCC 14398
*Vibrio marinofulvus* ATCC 14395
*Vibrio marinovulgaris* ATCC 14394
*Vibrio mediterranei* ATCC 43341
*Vibrio metschnikovii* ATCC 7708
*Vibrio mytili* ATCC 51288
*Vibrio natriegens* ATCC 14048
*Vibrio navarrensis* ATCC 51183
*Vibrio nereis* ATCC 25917
*Vibrio nigripulchritudo* ATCC 27043
*Vibrio ordalii* ATCC 33509
*Vibrio orientalis* ATCC 33933
*Vibrio pectenicida* ATCC 700783
*Vibrio pelagius* ATCC 33504
*Vibrio penaeicida* ATCC 51841
*Vibrio ponticus* ATCC 14391
*Vibrio proteolyticus* ATCC 53559
*Vibrio psychroerythrus* ATCC 27364
*Vibrio salmonicida* ATCC 43839
*Vibrio shiloii* ATCC BAA-91
*Vibrio splendidus* ATCC 33125
*Vibrio tyrosinaticus* ATCC 19378
*Vibrio viscosus* ATCC BAA-105
*Vibrio wodanis* ATCC BAA-104
*Beneckea pelagia* ATCC 25916
*Listonella anguillarum* ATCC 19264

*Beneckea pelagia* and *Listonella anguillarum* are closely related to the *Vibrio* bacteria, and some strains thereof are classified into *Vibrio* bacteria according to the current classification (Thompson F.L. et al., Microbiol. Mol. Biol. Rev., 23, 403-431 (2004) and Macian M.C. et al., Syst. Appl. Microbiol., 23, 373-375 (2000)). Therefore, such bacteria can also be used in the present invention as the *Vibrio* bacteria.

It is especially preferable to use *Vibrio natriegens. Vibrio natriegens* is an oceanic facultative anaerobic bacterium belonging to the family *Vibrionaceae* of Υ-*Proteobacteria,* and it was isolated on 1958 as an uronic acid-oxidizing bacterium (Payne, W.J., J. Bacteriology, 76, 301 (1958)). At first, the bacterium was considered to belong to the genus *Pseudomonas,* which genus is also of Υ-*Proteobacteria,* but the bacterium was re-classified into the genus *Beneckea* and then incorporated into the genus *Vibrio* along with other bacteria belonging to the genus *Beneckea.* That bacterium is classified as Biosafety level 1 in ATCC, and classified as Risk Group 1 (German classification) in the German National Resource Center for Biological Material (DSMZ), and the pathogenicity is not known for the bacterium.

As *Vibrio natriegens,* the *Vibrio natriegens* IFO 15636 strain (ATCC 14048) can be used.

The *Vibrio* bacteria mentioned above are available from, for example, American Type Culture Collection (Address: P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

The *Vibrio* bacteria can grow well under high osmotic pressure conditions during the period where the produced substance is accumulated at a high level at a later stage of amino acid fermentation, or under high osmotic pressure conditions due to a high sugar concentration, in contrast to microorganisms that have been conventionally used for production of an L-amino acid (such as *Escherichia coli* and coryneform bacteria), which cannot sufficiently grow under such conditions as mentioned above. It is preferable that the "high osmotic pressure" referred to here is, for example, not lower than 925 mOsm, preferably not lower than 1100 mOsm, more preferably not lower than 1500 mOsm. The maximum osmotic pressure is not particularly limited so long as the amino acid fermentation is possible, and is, for example, 2000 mOsm. In addition, the expression "the bacteria can grow well under high osmotic pressure" means that the growth rate is maintained to be, for example, higher than 50% of the maximum growth rate at 1100 mOsm, at which the growth rate of the *E. coli* wild-type strain, for example, MG1655 strain (ATCC 47076), decreases to 50% or less of the maximum growth rate, preferably not lower than about 90% of the maximum growth rate.

Methods for imparting an L-amino acid-producing ability to such bacteria as mentioned above and methods for enhancing an L-amino acid-producing ability of such bacteria as mentioned above are described below.

To impart an ability to produce an L-amino acid, methods conventionally employed in the breeding of coryneform bacteria or bacteria of the genus *Escherichia* (see "Amino Acid Fermentation", Gakkai Shuppan Center (Ltd.), 1st Edition, published May 30, 1986, pp.77-100) can be used. Such methods include acquiring an auxotrophic mutant, an L-amino acid analogue-resistant strain, or a metabolic regulation mutant, or constructing a recombinant strain in which an L-amino acid biosynthetic enzyme is overexpressed. In the breeding of L-amino acid-producing bacteria, the above-described property(s) such as auxotrophy, analogue resistance, and metabolic regulation mutation can be imparted alone or in combinations of two or more. The expression of L-amino acid biosynthesis enzyme(s) can be enhanced alone or in combinations of two or more. Furthermore, imparting such properties as an auxotrophy, analogue resistance, or metabolic regulation mutation can be combined with enhancing the biosynthetic enzymes.

An auxotrophic mutant strain, L-amino acid analogue-resistant strain, or metabolic regulation mutant strain with the ability to produce an L-amino acid can be obtained by subjecting a parent or wild-type strain to conventional mutagenesis, such as exposure to X-rays or UV irradiation, or treatment with a mutagen such as N-methyl-N'-nitro-N-nitrosoguanidine, and then selecting those which exhibit autotrophy, analogue resistance, or a metabolic regulation mutation and which also have the ability to produce an L-amino acid.

Moreover, the L-amino acid-producing ability can also be imparted or enhanced by increasing enzymatic activity by gene recombination. An example of the method for increasing enzymatic activity includes modifying the bacterium so that expression of a gene coding for an enzyme involved in biosynthesis of an L-amino acid is enhanced. Gene expression can also be increased by introducing an amplification plasmid prepared by introducing a DNA fragment containing the gene into an appropriate plasmid, for example, a plasmid containing at least a gene responsible for replication and proliferation of the plasmid in the microorganism, increasing the copy number of the gene on the chromosome by conjugation, transfer, or the like, or introducing a mutation into the promoter region of the gene (refer to International Publication WO95/34672).

When an objective gene is introduced into the aforementioned amplification plasmid or chromosome, any promoter can be used to express the gene so long as the chosen promoter functions in bacteria belonging to the *Enterobacteriaceae* bacteria. The promoter can be the native promoter for the gene, or a modified promoter. The expression of a gene can also be controlled by suitably choosing a promoter that strongly functions in the *Enterobacteriaceae* bacteria, or by making the -35 and -10 regions of the promoter closer to the consensus sequence. These methods for enhancing expression of enzyme genes are fully described in International Publication WO00/18935, European Patent Laid-open No. 1010755, and so forth.

Specific methods for imparting an L-amino acid-producing ability to bacteria and bacteria imparted with L-amino acid-producing ability are exemplified below.

### L-Threonine-producing bacteria

Preferred examples of microorganisms having L-threonine-producing ability include bacteria in which one or more activities of L-threonine biosynthetic enzymes are enhanced. Examples of L-threonine biosynthetic enzymes include aspartokinase III *(lysC),* aspartate semialdehyde dehydrogenase *(asd),* aspartokinase I (thrA), homoserine kinase *(thrB),* threonine synthase *(thrC),* and aspartate aminotransferase (aspartate transaminase) *(aspC).* The names of the genes coding for the respective enzymes are mentioned in the parentheses after the names of the enzymes (the same shall apply throughout this specification). Among these enzymes, aspartate semialdehyde dehydrogenase, aspartokinase I, homoserine kinase, aspartate aminotransferase, and threonine synthase are particularly preferred examples. The genes coding for the L-threonine biosynthetic enzymes can be introduced into an *Escherichia* bacterium which has a reduced ability to decompose threonine. An example of such an *Escherichia* bacterium having a reduced ability to decompose threonine is the TDH6 strain, which is deficient in threonine dehydrogenase activity (Japanese Patent Laid-open (Kokai) No. 2001-346578).

The enzymatic activities of the L-threonine biosynthetic enzymes are inhibited by the endproduct, L-threonine. Therefore, for constructing L-threonine-producing strains, it is desirable that the genes for the L-threonine biosynthetic enzymes are modified so that the enzymes are desensitized to feedback inhibition by L-threonine. The aforementioned *thrA, thrB,* and *thrC* genes constitute the threonine operon, which forms an attenuator structure. The expression of the threonine operon is inhibited by isoleucine and threonine in the culture medium and also suppressed by attenuation. Therefore, the threonine operon can be modified by removing the leader sequence in the attenuation region or the attenuator (refer to Lynn, S.P., Burton, W.S., Donohue, T.J., Gould, R.M., Gumport, R.L, and Gardner, J.F., J. Mol. Biol. 194:59-69 (1987); WO02/26993 WO2005/049808).

The native promoter of the threonine operon is present upstream of the threonine operon, and can be replaced with a non-native promoter (refer to WO98/04715), or a threonine operon which has been modified so that expression of the threonine biosynthesis genes is controlled by the repressor and promoter of λ-phage can be constructed (European Patent No. 0593792). Furthermore, in order to modify a bacterium so that it is desensitized to feedback inhibition by L-threonine, a strain resistant to α-amino-β-hydroxyisovaleric acid (AHV) can be selected.

It is preferred that the copy number of the threonine operon that is modified to be desensitized to feedback inhibition by L-threonine is increased in a host, or the expression amount of the threonine operon is increased by ligating it to a potent promoter. The copy number can also be increased by, besides amplification using a plasmid, transferring the threonine operon to a genome by using a transposon, Mu-phage, or the like.

Other than increasing expression of the L-threonine biosynthetic genes, expression of the genes involved in the glycolytic pathway, TCA cycle, or respiratory chain, the genes that regulate the expression of these genes, or the genes involved in sugar uptake can also be preferably increased. Examples of such genes effective on L-threonine production include the genes encoding transhydrogenase (pntAB, European Patent No. 733712), phosphoenolpyruvate carboxylase *(ppc,* WO95/06114), phosphoenolpyruvate synthase *(pps,* European Patent No. 877090), and a gene encoding pyruvate carboxylase *(pyc)* from a coryneform bacterium or a *Bacillus* bacterium (WO99/18228, European Patent Laid-open No. 1092776).

It is also preferred that expression of a gene that imparts resistance to L-threonine or L-homoserine is enhanced, or resistance to L-threonine or L-homoserine is imparted to the host. Examples of such resistance genes include the *rhtA* gene (Res. Microbiol., 154:123-135 (2003)), *rhtB* gene (European Patent Laid-open No. 0994190), *rhtC* gene (European Patent Laid-open No. 1013765), and *yfiK* and *yeaS* genes (European Patent Laid-open No. 1016710). The methods for imparting L-threonine resistance to a host are described in European Patent Laid-open No. 0994190 and WO90/04636.

Examples of L-threonine-producing bacteria and parent strains for deriving them include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No. 5,631,157), *E. coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E*. *coli* MG442 (Gusyatiner et al., Genetika (in Russian), 14, 947-956 (1978)), *E*. *coli* VL643 and VL2055 (European Patent Laid-open No. 1149911), and so forth.

The TDH-6 strain is deficient in the *thrC* gene, and sucrose-assimilative, and the *ilvA* gene thereof has a leaky mutation. This strain also has a mutation in the rhtA gene, which imparts resistance to high concentration of threonine or homoserine. The B-3996 strain contains the plasmid pVIC40, which was obtained by inserting the *thrA*BC* operon, including a mutant *thrA* gene, into the RSF1010-derived vector. This mutant *thrA* gene encodes aspartokinase homoserine dehydrogenase I which is substantially desensitized to feedback inhibition by threonine. The B-3996 strain was deposited on November 19, 1987 in the All-Union Scientific Center of Antibiotics (Nagatinskaya Street 3-A, 117105 Moscow, Russia) under the accession number RIA 1867. The strain was also deposited at the Russian National Collection of Industrial Microorganisms (VKPM) (1 Dorozhny proezd., 1 Moscow 117545, Russia) on April 7, 1987 under the accession number VKPM B-3996.

*E. coli* VKPM B-5318 (European Patent No. 0593792) can also be used as an L-threonine-producing bacterium or a parent strain for deriving it. The B-5318 strain is prototrophic with regard to isoleucine, and a temperature-sensitive lambda-phage Cl repressor and PR promoter replace the regulatory region of the threonine operon in the plasmid pVIC40. The VKPM B-5318 strain was deposited as an international deposit at the Russian National Collection of Industrial Microorganisms (VKPM) (1 Dorozhny proezd., 1 Moscow 117545, Russia) on May 3, 1990 under the accession number of VKPM B-5318.

The *thrA* gene coding for aspartokinase homoserine dehydrogenase I of *Escherichia coli* has been elucidated (nucleotide numbers 337 to 2799, GenBank accession NC_000913.2, gi: 49175990). The *thrA* gene is located between the *thrL* and *thrB* genes on the chromosome of *E*. *coli* K-12. The *thrB* gene coding for homoserine kinase of *Escherichia coli* has been elucidated (nucleotide numbers 2801 to 3733, GenBank accession NC_000913.2, gi: 49175990). The *thrB* gene is located between the *thrA* and *thrC* genes on the chromosome of *E. coli* K-12. The *thrC* gene coding for threonine synthase of *Escherichia coli* has been elucidated (nucleotide numbers 3734 to 5020, GenBank accession NC_000913.2, gi: 49175990). The *thrC* gene is located between the *thrB* gene and the *yaaX* open reading frame on the chromosome of *E. coli* K-12. All these three genes function as a single threonine operon. To increase expression of the threonine operon, the attenuator region, which affects the transcription, is preferably removed from the operon (WO2005/049808, WO2003/097839).

A mutant *thrA* gene coding for aspartokinase homoserine dehydrogenase I resistant to feedback inhibition by threonine, as well as the *thrB* and *thrC* genes can be obtained as a single operon from the well-known pVIC40 plasmid, which is present in the threonine-producing *E*. *coli* strain VKPM B-3996. The plasmid pVIC40 is described in detail in U.S. Patent No. 5,705,371.

The rhtA gene is present at 18 min on the *E*. *coli* chromosome close to the *glnHPQ* operon, which encodes components of the glutamine transport system. The rhtA gene is identical to ORF1 *(ybiF* gene, nucleotide numbers 764 to 1651, GenBank accession number AAA218541, gi:440181) and is located between the pexB and *ompX* genes. The unit expressing the protein encoded by the ORP1 has been designated the *rhtA* gene *(rht:* resistance to homoserine and threonine). Also, it was revealed that the rhtA23 mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, European Patent Laid-open No. 1013765).

The *asd* gene coding for aspartate semialdehyde dehydrogenase of *E*. *coli* has already been elucidated (nucleotide numbers 3572511 to 3571408, GenBank accession NC_000913.1, gi:16131307), and can be obtained by PCR (refer to White, T.J. et al., Trends Genet, 5, 185 (1989)) utilizing primers prepared on the basis of the nucleotide sequence of the gene. The *asd* genes of other microorganisms can also be obtained in a similar manner.

Also, the *aspC* gene coding for aspartate aminotransferase of *E*. *coli* has already been elucidated (nucleotide numbers 983742 to 984932, GenBank accession NC_000913.1, gi:16128895), and can be obtained by PCR. The *aspC* genes of other microorganisms can also be obtained in a similar manner.

### L-Lysine-producing bacteria

Examples of L-lysine-producing bacteria belonging to the genus *Escherichia* include mutants having resistance to an L-lysine analogue. L-Lysine analogues inhibit the growth of *Escherichia* bacteria, but this inhibition is fully or partially eliminated when L-lysine is present in the medium. Examples of these L-lysine analogues include, but are not limited to, oxalysine, lysine hydroxamate, S-(2-aminoethyl)-L-cysteine (AEC), Υ-methyllysine, α-chlorocaprolactam, and so forth. Mutants having resistance to these lysine analogues can be obtained by subjecting *Escherichia* bacteria to conventional artificial mutagenesis treatments. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, aspartokinase is desensitized to the feedback inhibition by L-lysine.

Examples of L-lysine-producing bacteria and parent strains for deriving them also include strains in which expression of one or more genes encoding an L-lysine biosynthetic enzyme are enhanced. Examples of such enzymes include, but are not limited to, dihydrodipicolinate synthase *(dapA),* aspartokinase *(lysC),* dihydrodipicolinate reductase *(dapB),* diaminopimelate decarboxylase *(lysA),* diaminopimelate dehydrogenase (ddh) (U.S. Patent No. 6,040,160), phosphoenolpyruvate carboxylase *(ppc),* aspartate semialdehyde dehydrogenase *(asd),* succinyl diaminopimelate deacylase *(dapE),* and aspartase *(aspA)* (European Patent Laid-open No. 1253195). Among these enzymes, dihydrodipicolinate reductase, diaminopimelate decarboxylase, diaminopimelate dehydrogenase, phosphoenolpyruvate carboxylase, aspartate aminotransferase, diaminopimelate epimerase, and aspartate semialdehyde dehydrogenase are particularly preferred. In addition, the parent strains can express increased levels of the gene involved in energy efficiency *(cyo)* (European Patent Laid-open No. 1170376), the genes encoding nicotinamide nucleotide transhydrogenase *(pntAB)* (U.S. Patent No. 5,830,716), the *ybjE* gene, which is a gene for L-lysine secretion (WO2005/073390), or combinations of these.

Examples of L-lysine-producing bacteria and parent strains for deriving them also include strains in which the activity of an enzyme that catalyzes a reaction for generating a compound other than L-lysine, which reaction branches away from the biosynthetic pathway of L-lysine, is reduced or eliminated. Examples of the enzyme that catalyzes a reaction for generating a compound other than L-lysine, which reaction branches away from the biosynthetic pathway of L-lysine, include homoserine dehydrogenase, lysine decarboxylase (U.S. Patent No. 5,827,698), and the malic enzyme (WO2005/010175).

Preferred examples of L-lysine-producing bacteria include *Escherichia coli* WC196ΔcadAΔldc/pCABD2 (WO2006/078039). This strain was constructed from the WC196 strain by disrupting *cadA* and *ldcC* genes, which encode lysine decarboxylase, and introducing the plasmid pCABD2 containing lysine biosynthetic genes (U.S. Patent No. 6,040,160) into the resulting strain. The WC196 strain was bred from the W3110 strain, which was derived from *Escherichia coli* K-12, by replacing the wild type *lysC* gene on the chromosome of the W3110 strain with a mutant *lysC* gene encoding a mutant aspartokinase III in which threonine at position 352 was replaced with isoleucine, resulting in desensitization of the feedback inhibition thereof by L-lysine (U.S. Patent No. 5,661,012), and conferring AEC resistance to the resulting strain (U.S. Patent No. 5,827,698). The WC196 strain was designated *Escherichia coli* AJ13069, deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994, and assigned an accession number of FERM P-14690. Then, it was converted to an international deposit under the provisions of the Budapest Treaty on September 29, 1995, and assigned an accession number of FERM BP-5252 (U.S. Patent No. 5,827,698). The WC196ΔcadAΔldc strain itself is also a preferred L-lysine-producing bacterium. The WC196ΔcadAΔldc was designated AJ110692, and deposited at the independent administrative agency, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on October 7, 2008 as an international deposit and assigned an accession number of FERM BP-11027.

The plasmid pCABD2 contains a mutant *dapA* gene derived from *Escherichia coli* and coding for a dihydrodipicolinate synthase (DDPS) having a mutation for desensitization to the feedback inhibition by L-lysine, a mutant *lysC* gene derived from *Escherichia coli* and coding for aspartokinase III having a mutation for desensitization to the feedback inhibition by L-lysine, the *dapB* gene derived from *Escherichia coli* and coding for dihydrodipicolinate reductase, and the *ddh* gene derived from *Brevibacterium lactofermentum* and coding for diaminopimelate dehydrogenase (WO95/16042 and WO01/53459).

As an L-methionine-producing bacterium, an *Escherichia* bacterium deficient in the repressor of the L-methionine biosynthesis system *(metJ)* and having enhanced intracellular homoserine transsuccinylase activity *(metA),* or having attenuated S-adenosylmethionine synthase activity *(metK)* can be used (Japanese Patent No. 04110641).

L-Tryptophan, L-phenylalanine, and L-tyrosine are all aromatic amino acids and share a common biosynthesis pathway. Examples of the genes encoding the biosynthetic enzymes for aromatic amino acids include genes of deoxyarabino-heptulosonate phosphate synthase *(aroG),* chorismate mutase/prephenate dehydratase *(pheA),* 3-dehydroquinate synthase *(aroB),* shikimate dehydrogenase *(aroE),* shikimate kinase (aroL), 5-enolpyruvylshikimate-3-phosphate synthase *(aroA),* and chorismate synthase *(aroC)* (European Patent Laid-open No. 763127). It is known that these genes are regulated by the tyrosine repressor *(tyrR),* and so activity of an aromatic amino acid biosynthetic enzyme can also be increased by deleting the *tyrR* gene (see European Patent No. 763127).

Furthermore, because 3-deoxy-D-arabinoheptulosonate-7-phosphate synthetase *(aroF, aroG)* is inhibited by feedback inhibition by aromatic amino acids, the enzyme can be modified so as to be desensitized to the feedback inhibition. An aromatic amino acid-producing bacterium can be obtained, for example, by introducing a mutant *aroF* gene coding for a protein in which the L-aspartic acid at position 147 or the L-serine at position 181, as counted from the N-terminus, is replaced by another amino acid, or by introducing a mutant *aroG* gene coding for a protein in which single amino acid residue of the L-aspartic acid at position 146, the L-methionine at position 147, the L-proline at position 150, or the L-alanine at position 202, or two amino acid residues of the L-methionine at position 157 and the L-alanine at position 219, as counted from the N-terminus, are replaced by other amino acid(s) (European Patent Laid-open No. 0488424). Furthermore, because the chorismate mutase/prephenate dehydratase is also inhibited by feedback inhibition by aromatic amino acids, it can be modified so as to be desensitized to the feedback inhibition.

Because the aromatic amino acids share a common biosynthesis pathway, a strain in which a biosynthesis system specific to an aromatic amino acid other than the objective L-amino acid is attenuated is preferably used. For example, a strain that efficiently produces an objective L-amino acid can be obtained by attenuating biosynthesis systems specific to L-phenylalanine or L-tyrosine when the objective amino acid is L-tryptophan, or by attenuating biosynthesis systems specific to L-tryptophan or L-tyrosine when the objective amino acid is L-phenylalanine. Attenuation of a biosynthesis system can be attained by introducing a mutation into a gene coding for an enzyme of the biosynthesis system, or obtaining a strain requiring an L-amino acid synthesized by the biosynthesis system to be attenuated using a synthetic medium containing the L-amino acid (U.S. Patent No. 4,371,614).

### L-Tryptophan-producing bacteria

Examples of L-tryptophan-producing bacteria and parent strains for deriving them include, but are not limited to, *Escherichia* bacteria strains, such as *E. coli* JP4735/pMU3028 (DSM10122) and *E. coli* JP6015/pMU91 (DSM10123), which lack tryptophanyl-tRNA synthetase encoded by a mutant *trpS* gene (U.S. Patent No. 5,756,345), *E. coli* AGX17 (pGX44) (NRRL B-12263) and *E. coli* AGX6 (pGX50) aroP (NRRL B-12264), which lack tryptophanase (U.S. Patent No. 4,371,614), and *E. coli* AGX17/pGX50,pACKG4-pps, in which phosphoenolpyruvate-producing ability is increased (WO97/08333, U.S. Patent No. 6,319,696). L-Tryptophan-producing bacteria belonging to the genus *Escherichia* with increased activity of the protein encoded by the *yedA* gene or the *yddG* gene can also be used (U.S. Patent Published Application Nos. 2003/0148473 and 2003/0157667).

Examples of L-tryptophan-producing bacteria and parent strains for deriving them also include strains in which one or two or more activities of the enzymes selected form the following are increased: anthranilate synthase *(trpE),* phosphoglycerate dehydrogenase (serA), 3-deoxy-D-arabinoheptulosonate-7-phosphate synthase *(aroG), 3-*dehydroquinate synthase *(aroB),* shikimate dehydrogenase *(aroE),* shikimate kinase *(aroL),* 5-enolpyruvylshikimate-3-phosphate synthase *(aroA),* chorismate synthase (aroC), prephenate dehydratase, chorismate mutase, and tryptophan synthase (trpAB). The anthranilate synthase and phosphoglycerate dehydrogenase are both inhibited by feedback inhibition by L-tryptophan and L-serine, and therefore a mutation desensitizing the enzymes for the feedback inhibition can be introduced into these enzymes. Specific examples of strains having such a mutation include *E. coli* SV164(trpE8), which harbors desensitized anthranilate synthase, and SV164 (pGH5) obtained by introducing the plasmid pGH5 containing a mutant serA gene encoding feedback inhibition-desensitized phosphoglycerate dehydrogenase into the *E*. *coli* SV164.

The aforementioned *E*. *coli* SV164(trpE8) is a strain obtained by introducing a mutant *trpE* gene coding for anthranilate synthase desensitized to the feedback inhibition by L-tryptophan into a trpE-deficient strain, *Escherichia coli* KB862 (DSM7196) (WO94/08031, Japanese Patent Laid-open (Kohyo) No. 7-507693). The *E*. *coli* SV164(pGH5) strain is a strain obtained by introducing a plasmid pGH5 (WO94/08031) containing a mutant serA5 gene coding for phosphoglycerate dehydrogenase desensitized to feedback inhibition by serine into the SV164 strain. The *E*. *coli* SV164(pGH5) strain produces not only L-tryptophan but also L-serine (U.S, Patent No. 7,045,320).

The aforementioned *E*. *coli* KB862 strain was designated AJ13828 and deposited on December 21, 2000 at National Institute of Bioscience and Human Technology of Agency of Industrial Science and Technology (currently independent administrative agency, National Institute of Advanced Industrial Science and Technology, International Patent Organism Depositary, Tsukuba Central 6, 1-1, Higashi 1-Chome, Tsukuba-shi, Ibaraki-ken, 305-8566, Japan) as an international deposit under the provisions of the Budapest Treaty, and assigned an accession number of FERM BP-7405.

Examples of L-tryptophan-producing bacteria and parent strains for deriving them also include a strain which has enhanced activity of 3-phosphoserine phosphatase *(serB)* (U.S. Patent No. 4,371,614), a strain which has enhanced activity of phosphoenolpyruvate carboxykinase *(pckA)* (WO2004/090125), and a strain which constitutively express maleate synthase-isocitrate lyase-isocitrate dehydrogenasekinase/phosphatase operon (ace operon), or of which expression of this operon is enhanced (WO2005/103275).

Examples of L-tryptophan-producing bacteria and parent strains for deriving them also include strains which have been transformed with the tryptophan operon containing a gene encoding inhibition-desensitized anthranilate synthase (Japanese Patent Laid-open Nos. 57-71397, 62-244382, U.S. Patent No. 4,371,614). Moreover, L-tryptophan-producing ability can be imparted by increasing expression of a gene coding for tryptophan synthase in the tryptophan operon *(trpBA).* Tryptophan synthase consists of **α** and **β** subunits which are encoded by the *trpA* and *trpB* genes, respectively. In addition, L-tryptophan-producing ability can be improved by increasing expression of the isocitrate lyase-malate synthase operon (WO2005/103275).

### L-Phenylalanine-producing bacteria

Examples of L-phenylalanine-producing bacteria and parent strains for deriving them include, but are not limited to, strains belonging to the genus *Escherichia,* such as *E. coli* AJ12479 (FERM BP-4796) (European Patent Laid-open No. 1484410), *E. coli* AJ12739 deficient in chorismate mutase-prephenate dehydratase and tyrocine repressor (tyrA: :Tn10, tyrR) (VKPM B-8197), *E*. *coli* HW1089 (ATCC 55371) harboring a mutant *pheA34* gene coding for chorismate mutase-prephenate dehydratase desensitized to feedback inhibition (U.S. Patent No. 5,354,672), *E. coli* MWEC101-b (KR8903681), *E. coli* NRRL B-12141, NRRL B-12145, NRRL B-12146, and NRRL B-12147 (U.S. Patent No. 4,407,952). As a parent strain, *E*. *coli* K-12 [W3110 (tyrA)/pPHAB] (FERM BP-3566) harboring a gene coding for chorismate mutase-prephenate dehydratase desensitized to the feedback inhibition, *E*. *coli* K-12 [W3110 (tyrA)/pPHAD] (FERM BP-12659), *E*. *coli* K-12 [W3110 (tyrA)/pPHATerm] (FERM BP-12662) and *E*. *coli* K-12 [W3110 (tyrA)/pBR-aroG4, pACMAB] named as AJ12604 (FERM BP-3579) can also be used (European Patent No. 488424). Furthermore, L-phenylalanine-producing bacteria belonging to the genus *Escherichia* with an increased activity of the protein encoded by the *yedA* gene or the *yddG* gene can also be used (U.S. Patent Published Application Nos. 2003/0148473 and 2003/0157667).

As for phenylalanine-producing bacteria, strains efficiently producing L-phenylalanine can also be obtained by such modification that bacteria incorporate by-products into cells, for example, by increasing expression amount of the L-tryptophan uptake gene, *tnaB* or *mtr,* or the L-tyrosine uptake gene, *tyrP* (European Patent No. 1484410).

### L-Tyrosine-producing bacteria

Examples of tyrosine-producing bacteria include *Escherichia* bacteria having a desensitized type prephenate dehydratase gene (tyrA), of which the product is not inhibited by tyrosine (European Patent Laid-open No. 1616940).

When the bacterium used for the present invention is bred by gene recombination, the genes to be used are not limited to genes having the genetic information described above or genes having known sequences, but also include variants of the genes, namely, genes having conservative mutations, such as homologues of the genes or artificially modified genes, can also be used so long as the functions of the encoded proteins are not degraded. That is, they can be genes encoding a protein having a known amino acid sequence, but including substitution, deletion, insertion, addition, or the like of one or several amino acid residues at one or several positions.

Although the number of the "one or several" referred to herein can differ depending on the position of amino acid residues in the three-dimensional structure of the protein or the types of amino acid residues, specifically, it can be preferably 1 to 10, more preferably 1 to 5, still more preferably 1 to 3, particularly preferably 1 or 2. The conservative mutation is typically a conservative substitution. The conservative substitution is a mutation wherein substitution takes place mutually among Phe, Trp, and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Substitutions considered conservative substitutions include, specifically, substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned amino acid substitution, deletion, insertion, addition, inversion, or the like can be a result of a naturally-occurring mutation or a variation due to an individual difference or difference of species of a microorganism from which the genes are derived (mutant or variant). Such genes can be obtained by, for example, modifying a known nucleotide sequence of a gene by site-specific mutagenesis so that the amino acid residues at the specific sites of the encoded protein include substitution, deletion, insertion, or addition of amino acid residues.

Furthermore, such genes having conservative mutation(s) as described above can encode a protein having a homology of 80% or more, preferably 90% or more, more preferably 95% or more, particularly preferably 97% or more, to the entire encoded amino acid sequence and having a function equivalent to that of the wild-type protein. In this specification, "homology" can mean "identity".
Moreover, codons in the gene sequences can be replaced with other codons which are easily used in the host into which the genes are introduced.

The genes having conservative mutation(s) can be obtained by methods usually used in mutagenesis treatments such as treatments with mutagenesis agents.

Furthermore, the genes can be a DNA which can hybridize with a complementary sequence of a known gene sequence or a probe which can be prepared from such a complementary sequence under stringent conditions and encodes a protein having a function equivalent to that of the known gene product. The "stringent conditions" referred to here are conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, particularly preferably not less than 97% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

As the probe, a part of a complementary sequence of a gene can also be used. Such a probe can be prepared by PCR using oligonucleotides prepared on the basis of a known gene sequence as primers and a DNA fragment containing the nucleotide sequences as a template. For example, when a DNA fragment having a length of about 300 bp is used as the probe, the washing conditions of hybridization can be 50°C, 2 x SSC and 0.1% SDS.

The aforementioned descriptions concerning variants of genes are similarly applied to the *ydgT* gene described below and the other genes mentioned in this specification.

The bacterium used for the present invention is a bacterium having glycerol-assimilating ability, and it can be a bacterium inherently having glycerol-assimilating ability, or a recombinant strain imparted with glycerol-assimilating ability.

The L-amino acid-producing bacterium of the present invention can have been modified so that glycerol-assimilating ability thereof is enhanced. The glycerol-assimilating ability can be imparted or enhanced by modifying a gene involved in the glycerol metabolism.

In order to enhance glycerol-assimilating ability, expression of the *glpR* gene (European Patent No. 1715056) can be attenuated, or expression of a glycerol metabolism gene such as *glpA, glpB, glpC, glpD, glpE, glpF, glpG, glpK, glpQ, glpT, glpX, tpiA, gldA, dhaK, dhaL, dhaM, dhaR, fsa* and *talC* genes (European Patent Laid-open No. 1715055) can be enhanced. In particular, in order to enhance glycerol-assimilating property, it is preferred that expression of the glycerol dehydrogenase gene *(gldA),* dihydroxyacetone kinase gene *(dhaKLM, dak),* and fructose-6-phosphate aldolase gene *(fsaB)* is enhanced (WO2008/102861).
Furthermore, as for glycerol kinase *(glpK),* it is preferable to use a desensitized type *glpK* gene of which the product is desensitized to the feedback inhibition by fructose-1,6-phosphate (WO2008/081959, WO2008/107277).

### <1-2> Reduction of activity of Cnu protein

Hereafter, the modification for reducing the activity of the Cnu protein is explained.
The Cnu protein is encoded by the *ydgT* gene (synonyms: Cnu, ECK1621, b1625). Specific examples include, as the *ydgT* gene of *Escherichia coli,* a gene having the nucleotide sequence shown in SEQ ID NO: 13, which locates at the nucleotide numbers 1702973 to 1703188 of the genome sequence of *Escherichia coli* (GenBank Accession No. U00096). The amino acid sequence encoded by this gene is shown in SEQ ID NO: 14.
The *ydgT* gene product is also known as B1625, YdgT, or H-NS- and StpA-binding protein, and it has been reported that the Cnu protein binds to the replication origin oriC (Kim, M.S. et al., J. Bacteriol., 187:6998-7008 (2005)).
In the present invention, the "activity of the Cnu protein" is such a property that when the activity is reduced in a bacterium, for example, when amount of the Cnu protein in the cell is reduced, an L-amino acid-producing ability, for example, L-lysine-producing ability, is improved compared with that of a non-modified strain, or proliferation rate or glycerol-assimilating ability of the bacterium is improved.

Examples of the Cnu protein include, for example as for *Escherichia coli,* a protein having the amino acid sequence of SEQ ID NO: 14. However, the Cnu protein can also be a protein having the amino acid sequence, but including a conservative mutation(s) so long as the protein has the activity of the Cnu protein. That is, the Cnu protein can be a protein having the amino acid sequence of SEQ ID NO: 14, but including substitution, deletion, insertion, addition, or the like of one or several amino acid residues.

Although the number of the "one or several" referred to herein can differ depending on the position of amino acid residues in the three-dimensional structure or the types of amino acid residues of the protein, specifically, it can be, for example, 1 to 20, preferably 1 to 10, more preferably 1 to 5. The conservative mutation is typically a conservative substitution. The conservative substitution is a mutation wherein substitution takes place mutually among Phe, Trp, and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Substitutions considered conservative substitutions include, specifically, substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned amino acid substitution, deletion, insertion, addition, inversion, or the like can be a result of a naturally-occurring mutation or a variation due to an individual difference or difference of species of a microorganism from which the genes are derived (mutant or variant). Such genes can be obtained by, for example, modifying a known nucleotide sequence of a gene by site-specific mutagenesis so that the amino acid residues at the specific sites of the encoded protein include substitution, deletion, insertion, or addition of amino acid residues.

The gene coding for the Cnu protein can be a DNA that is able to hybridize with a nucleotide sequence complementary to the nucleotide sequence of SEQ ID NO: 13 or a probe that can be prepared from the nucleotide sequence under stringent conditions, so long as it codes for the Cnu protein. The "stringent conditions" refer to conditions under which a so-called specific hybrid is formed, and a non-specific hybrid is not formed. Examples of the stringent conditions include those under which highly homologous DNAs hybridize to each other, for example, DNAs not less than 90% homologous, preferably not less than 95% homologous, more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

As the probe, a part of a sequence that is complementary to the gene can also be used. Such a probe can be prepared by PCR using oligonucleotides prepared on the basis of a known gene sequence as primers and a DNA fragment containing the nucleotide sequence as a template. Although length of the probe is appropriately chosen according to the conditions of the hybridization, it is usually 100 bp to 1 kbp. When a DNA fragment having a length of about 300 bp is used as the probe, the washing conditions of the hybridization can be, for example, 50°C, 2 x SSC and 0.1% SDS.

The phrase "modified so that the activity of the Cnu protein is reduced" means that the activity of the Cnu protein per cell of the bacterium has become lower than that of a non-modified strain, such as a wild-type strain of the bacterium belonging to the family *Enterobacteriaceae.* This means, for example, that the number of molecules of the Cnu protein per cell is decreased, or that the activity of the Cnu protein per molecule is decreased. The term "reduction" of the activity includes the complete disappearance of the activity.

The phrase "the activity of the Cnu protein is reduced" preferably means that activity of the Cnu protein is reduced to, for example, 50% or less, preferably 30% or less, more preferably 10% or less, of the activity of a non-modified strain, such as a wild-type strain. Examples of the wild-type strain of *Escherichia* bacterium as a control of the comparison include, for example, the *Escherichia coli* MG1655 strain, and so forth.

The modification for reducing the activity of the Cnu protein can be achieved by, specifically, deleting a part or all of the *ydgT* gene on a chromosome, or modifying an expression control sequence such as a promoter or the Shine-Dalgarno (SD) sequence of the gene. The reduction of expression includes reduction of transcription and reduction of translation. Furthermore, expression of the gene can also be reduced by modifying a non-translation region other than the expression control sequence. The entire gene as well as the sequences on both sides of the gene on the chromosome can also be deleted. Furthermore, the modification can also be attained by introducing an amino acid substitution (missense mutation), a stop codon (nonsense mutation), or a frame shift mutation which adds or deletes one or two nucleotides, into the coding region of the gene on the chromosome (Journal of Biological Chemistry, 272:8611-8617 (1997); Proceedings of the National Academy of Sciences, USA, 95:5511-5515 (1998); Journal of Biological Chemistry, 266, 20833-20839 (1991)).
Modifying a gene so that the function of the gene or the expression product thereof is decreased or deleted as described above is also called gene disruption. In the present invention, disruption of the gene of the Cnu protein is preferably performed by deleting the entire sequence or 30% or more, preferably 50% or more, more preferably 70% or more, of the promoter and/or the coding region of the gene.

Modification of the gene is preferably attained by gene recombination. Specific examples of the methods based on gene recombination include deleting a part or all of an expression control sequence such as a promoter region, a coding region, or a non-coding region of a target gene on the chromosome, inserting another sequence into these regions, and introducing a frame shift mutation, a nonsense mutation, or a missense mutation into these regions, by utilizing homologous recombination.

A modification of an expression control sequence is performed for preferably one or more nucleotides, more preferably two or more nucleotides, particularly preferably three or more nucleotides. When a coding region is deleted, the region to be deleted can be any of an N-terminus region, an internal region, or a C-terminus region, or even can be the entire coding region, so long as the function of the protein produced from the gene is decreased or deleted. Deletion of a longer region can usually more surely inactivate a target gene. Furthermore, it is preferred that reading frames upstream and downstream of the region to be deleted are not the same.

Also when another sequence is inserted into the coding region, such a sequence can be inserted into any region of the target gene, and insertion of a longer sequence can more surely inactivate the target gene. It is preferred that reading frames upstream and downstream of the insertion site are not the same. The other sequence is not particularly limited so long as a sequence of which insertion decreases or deletes the function of a protein encoded by the target gene is chosen, and examples include a transposon carrying an antibiotic resistance gene or a gene useful for L-amino acid production, and so forth.

A target gene on the chromosome can be modified as described above by, for example, preparing a deletion-type gene in which a partial sequence of the gene is deleted so that the deletion-type gene does not produce a protein that normally functions, and transforming a bacterium with a DNA containing the deletion-type gene to cause homologous recombination between the deletion-type gene and the target gene on the chromosome, and thereby substitute the deletion-type gene for the target gene on the chromosome. The protein encoded by the deletion-type gene has a conformation different from that of the wild-type protein, if it is even produced, and thus the function is reduced or deleted. Such gene disruption based on gene substitution utilizing homologous recombination has been already established, and there are a method called "Red-driven integration" (Datsenko, K.A, and Wanner, B.L., Proc. Natl. Acad. Sci. USA, 97:6640-6645 (2000)), a method using a linear DNA such as a method utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method using a plasmid containing a temperature sensitive replication origin, a method using a plasmid capable of conjugative transfer, a method utilizing a suicide vector not having replication origin in a host (U.S. Patent No. 6,303,383, Japanese Patent Laid-open (Kokai) No. 05-007491), and so forth.

Decrease of the transcription amount of the target gene can be confirmed by comparing the amount of mRNA transcribed from the target gene with that observed in a wild-type strain or non-modified strain. Examples of the method for evaluating mRNA amount include Northern hybridization, RT-PCR (Molecular Cloning, Cold Spring Harbor Laboratory Press, Cold Spring Harbor, USA, 2001), and so forth. Although the transcription amount can be decreased to any extent so long as it is decreased compared with that observed in a wild-type strain or non-modified strain, it is desirably decreased to, for example, at least 75% or less, 50% or less, 25% or less, or 10% or less, of that observed in a wild-type strain or non-modified strain, and it is particularly preferred that the gene is not expressed at all.

Decrease in amount of the protein encoded by the target gene can be confirmed by Western blotting using antibodies that bind to the protein (Molecular Cloning, Cold Spring Harbor Laboratory Press, Cold Spring Harbor, USA, 2001). Although the amount of the protein can be decreased to any extent so long as it is decreased compared with that observed in a wild-type strain or non-modified strain, it is desirably decreased to, for example, at least 75% or less, 50% or less, 25% or less, or 10% or less, of that observed in a wild-type strain or non-modified strain, and it is particularly preferred that the protein is not produced at all (the activity has completely disappeared).

A gene coding for the Cnu protein showing low activity can also be obtained by subjecting the *ydgT* gene to a mutation treatment.

Examples of the method for reducing the activity of the Cnu protein include, besides the aforementioned genetic manipulation techniques, for example, a method of treating an *Escherichia* bacterium with ultraviolet irradiation or a mutagen used for usual mutagenesis treatment such as N-methyl-N'-nitro-N-nitrosoguanidine (NTG) or nitrous acid, and selecting a strain showing reduced activity of the Cnu protein.

### <2> Method for producing L-amino acid of the present invention

In the present invention, a bacterium belonging to γ-*Proteobacteria,* having the ability to produce an L-amino acid, and modified so that the activity of the Cnu protein is reduced is cultured in a medium containing glycerol as a carbon source to produce and accumulate the L-amino acid in the medium, and the L-amino acid is collected from the medium. Examples of the L-amino acid include the aforementioned L-amino acids, especially aspartic acid type amino acids and aromatic amino acids, and among these, L-lysine is particularly preferred.

In the present invention, glycerol is used as a carbon source. Glycerol can be used at any concentration so long as a concentration suitable for production of the L-amino acid is chosen. When glycerol is used as a sole carbon source in the medium, it is preferably contained in the medium in an amount of about 0.1 to 50 w/v %, more preferably about 0.5 to 40 w/v %, particularly preferably about 1 to 30% w/v %. Glycerol can also be used in combination with other carbon sources such as glucose, fructose, sucrose, blackstrap molasses, and starch hydrolysate. In this case, although glycerol and other carbon sources can be mixed at an arbitrary ratio, it is desirable that the ratio of glycerol in the carbon source should be 10% by weight or more, more preferably 50% by weight or more, still more preferably 70% by weight or more. Preferred as the other carbon sources are saccharides such as glucose, fructose, sucrose, lactose, galactose, blackstrap molasses, starch hydrolysate, and a sugar solution obtained by hydrolysis of biomass, alcohols such as ethanol, and organic acids such as fumaric acid, citric acid and succinic acid. Among these, glucose is preferred.

Although initial concentration of glycerol at the time of starting the culture is preferably as described above, glycerol can be supplemented according to the consumption of glycerol during the culture.
Glycerol to be used can be pure glycerol or crude glycerol. Crude glycerol refers to industrially produced glycerol containing impurities. Crude glycerol is industrially produced by contacting oils with water under a high temperature and high pressure to hydrolyze them, or by the esterification reaction for biodiesel fuel production. Biodiesel fuel refers to fatty acid methyl esters produced from oils and methanol by a transesterification reaction, and crude glycerol is produced as a by-product of this reaction (refer to Fukuda, H., Kondo, A., and Noda, H., 2001, J. Biosci. Bioeng., 92, 405-416). In the biodiesel fuel production process, the alkaline catalyst method is used for the transesterification in many cases, and acids are added for neutralization. Therefore, crude glycerol of a purity of about 70 to 95% by weight, containing water and impurities, is produced. Crude glycerol produced in the biodiesel fuel production process contains, in addition to water, residual methanol and salts of alkali such as NaOH as a catalyst and an acid used for neutralization thereof such as H₂SO₄ as impurities. Although it depends on manufacturers and production methods, the amount of these salts and methanol can reach several percents. The crude glycerol preferably contains ions derived from the alkali and the acid used for neutralization thereof, such as sodium ions, potassium ions, chloride ions, and sulfate ions, in an amount of 2 to 7%, preferably 3 to 6%, more preferably 4 to 5.8%, based on the weight of the crude glycerol. Although methanol may not be contained as an impurity, it is preferably contained in an amount of 0.01% or less.

The crude glycerol can further contain trace amounts of metals, organic acids, phosphorus, fatty acids, and so forth. Examples of the organic acids to be contained include formic acid, acetic acid, and so forth, and although they may not be contained as impurities, they are preferably contained in an amount of 0.01% or less. As the trace metals contained in the crude glycerol, trace metals required for growth of the microorganism are preferred, and examples include, for example, magnesium, iron, calcium, manganese, copper, zinc, and so forth. Magnesium, iron, and calcium are preferably contained in an amount of 0.00001 to 0.1%, preferably 0.0005 to 0.1%, more preferably 0.004 to 0.05%, still more preferably 0.007 to 0.01%, in terms of the total amount based on the weight of the crude glycerol. Manganese, copper, and zinc are preferably contained in an amount of 0.000005 to 0.01%, preferably 0.000007 to 0.005%, more preferably 0.00001 to 0.001%, in terms of the total amount.

It is sufficient that the purity of glycerol in the crude glycerol is 10% or higher, and is preferably 50% or higher, more preferably 70% or higher, particularly preferably 80% or higher. As long as the contained amounts of impurities are within the aforementioned ranges, the purity of the glycerol can be 90% or higher.
When crude glycerol is used, the crude glycerol can be added to the medium according to the purity of the glycerol so that the amount of glycerol is within the concentration range described above. Both glycerol and crude glycerol can be added to the medium.

As the medium, ordinary media containing a nitrogen source, inorganic ions, and other organic components as required, in addition to the aforementioned carbon source, can be used. As the nitrogen source contained in the medium used in the present invention, ammonia, ammonium salts such as ammonium sulfate, ammonium carbonate, ammonium chloride, ammonium phosphate, ammonium acetate, and urea, nitrate salts, and so forth can be used. Ammonia gas or aqueous ammonia used for adjustment of pH can also be used as the nitrogen source. Peptone, yeast extract, meat extract, malt extract, corn steep liquor, soybean hydrolysate, and so forth can also be used as the nitrogen source. The medium can contain one of these nitrogen sources, or two or more of them. These nitrogen sources can be used in both starting medium and feed medium. Furthermore, the same nitrogen source can be used in both the starting medium and feed medium, or nitrogen source of the feed medium can be different from nitrogen source of the starting medium.

The medium used in the present invention preferably contains a phosphate source and a sulfur source in addition to the carbon source and the nitrogen source. As the phosphate source, potassium dihydrogen phosphate, dipotassium hydrogen phosphate, phosphate polymers such as pyrophosphoric acid and so forth can be utilized. Although the sulfur source can be any substance containing sulfur atoms, sulfate salts such as sulfates, thiosulfates, and sulfites, and sulfur-containing amino acids such as cysteine, cystine, and glutathione are desirable, and ammonium sulfate is especially desirable.

Furthermore, the medium can contain a growth-promoting factor (nutrient having a growth promoting effect) in addition to the carbon source, nitrogen source, and sulfur source. As the growth promoting factor, trace metals, amino acids, vitamins, nucleic acids as well as peptone, casamino acid, yeast extract, soybean protein degradation product, and so forth containing the foregoing substances can be used. Examples of the trace metals include iron, manganese, magnesium, calcium, and so forth. Examples of the vitamins include vitamin B₁, vitamin B₂, vitamin B₆, nicotinic acid, nicotinamide, vitamin B₁₂, and so forth. These growth-promoting factors can be contained in the starting medium or the feed medium.

Furthermore, when an auxotrophic mutant strain that requires an amino acid or the like for growth thereof is used, it is preferable to supplement a required nutrient to the medium. In particular, because the L-lysine biosynthetic pathway is often enhanced and L-lysine-degrading ability is often attenuated in L-lysine-producing bacteria that can be used for the present invention as described above, one or more substances selected from L-threonine, L-homoserine, L-isoleucine, and L-methionine are preferably added. The starting medium and the feed medium can have the same medium composition or different medium compositions. Furthermore, when the feed medium is fed at multiple steps, the composition of the feed medium fed at each of the steps can be the same or different.

The culture is preferably performed as aeration culture at a fermentation temperature of 20 to 45°C, particularly preferably 33 to 42°C. The culture is preferably performed with controlling the oxygen concentration to be about 5 to 50%, desirably about 10%. Furthermore, pH is preferably controlled to be 5 to 9 during the aeration culture. If pH of the medium is lowered during the culture, for example, calcium carbonate can be added, or the medium can be neutralized with an alkaline such as ammonia gas and aqueous ammonia. If culture is performed under such conditions as described above preferably for about 10 to 120 hours, a marked amount of L-amino acid is accumulated in the culture medium. Although the concentration of L-amino acid accumulated is not limited so long as it is higher than that observed with a wild-type strain, and it enables isolation and collection of the L-amino acid from the medium, it is desirably 50 g/L or higher, more desirably 75 g/L or higher, still more desirably 100 g/L or higher.

In the present invention, in order to keep the accumulation of L-amino acid to be a certain level or higher, culture of the microorganism can be carried out as separate seed culture and main culture. The seed culture can be carried out with shaking using a flask or the like or as batch culture, and the main culture can be carried out as fed-batch culture or continuous culture. Both the seed culture and main culture can be carried out as batch culture.

When the objective amino acid is a basic amino acid, the production can be performed by a method in which fermentation is performed by controlling pH of the medium during culture to be 6.5 to 9.0 and pH of the medium at the end of the culture to be 7.2 to 9.0 and controlling the pressure in the fermentation tank to be positive during the fermentation, or by supplying carbon dioxide gas or a mixed gas containing carbon dioxide gas to the medium to provide a culture period where the medium contains 2 g/L or more of bicarbonate ions and/or carbonate ions, so that these bicarbonate ions and/or carbonate ions act as counter ions of cations mainly consisting of a basic amino acid, and the objective basic amino acid is then collected (refer to Japanese Patent Laid-open (Kokai) No. 2002-065287).

The L-amino acid can be collected from fermentation broth by a combination of conventionally known methods such as ion-exchange resin method (Nagai, H. et al., Separation Science and Technology, 39(16), 3691-3710), precipitation, membrane separation (Japanese Patent Laid-open (Kokai) Nos. 9-164323 and 9-173792), crystallization (WO2008/078448, W02008/078646), and other methods. When the L-amino acid accumulates in the cells, the cells can be disrupted with, for example, ultrasonic waves or the like, and the L-amino acid can be collected by the ion exchange resin method or the like from the supernatant obtained by removing the cells from the cell-disrupted suspension by centrifugation.

The L-amino acid collected can contain bacterial cells, medium components, moisture, and by-product metabolites of the bacterium in addition to the objective L-amino acid. Purity of the collected L-amino acid can be 50% or higher, preferably 85% or higher, particularly preferably 95% or higher (Japanese Patent No. 1214636, U.S. Patent Nos. 5,431,933, 4,956,471, 4,777,051, 4,946,654, 5,840,358, 6,238,714, U.S. Patent Published Application No. 2005/0025878).

Furthermore, when L-amino acid precipitates in the medium, it can be collected by centrifugation, filtration, or the like. L-Amino acid precipitated in the medium and L-amino acid dissolved in the medium can be isolated together after the L-amino acid dissolved in the medium is crystallized.

Phenylalanine produced by the method of the present invention can be used for, for example, producing a lower alkyl ester of α-L-aspartyl-L-phenylalanine (also referred to as "aspartame"). Therefore, the present invention also provides a method for producing a lower alkyl ester of α-L-aspartyl-L-phenylalanine using L-phenylalanine as a starting material. This method comprises the step of synthesizing a lower alkyl ester of α-L-aspartyl-L-phenylalanine from L-phenylalanine produced by the aforementioned method of the present invention, and aspartic acid or a derivative thereof. Examples of the lower alkyl ester include methyl ester, ethyl ester, propyl ester, and so forth.

As the method of the present invention, the method for synthesizing a lower alkyl ester of α-L-aspartyl-L-phenylalanine from L-phenylalanine and aspartic acid or a derivative thereof is not particularly limited, so long as L-phenylalanine or a derivative thereof is used for the synthesis of the lower alkyl ester of α-L-aspartyl-L-phenylalanine. Specifically, for example, a lower alkyl ester of α-L-aspartyl-L-phenylalanine can be produced by the following method (U.S. Patent No. 3,786,039). L-Phenylalanine is esterified to obtain a lower alkyl ester of L-phenylalanine. The L-phenylalanine alkyl ester is reacted with an L-aspartic acid derivative of which β-carboxyl group is protected and α-carboxyl group is esterified and thereby activated. Examples of such a derivative include N-acyl-L-aspartic anhydride such as N-formyl-, N-carbobenzoxy-, or N-p-methoxycarbobenzoxy-L-aspartic anhydride. By this condensation reaction, a mixture of N-acyl-α-L-aspartyl-L-phenylalanine and N-acyl-β-L-aspartyl-L-phenylalanine is obtained. If the condensation reaction is performed in the presence of an organic acid of which acid dissociation constant at 37°C is 10⁻⁴ or less, the ratio of the α-isomer to the β-isomer in the mixture is increased (Japanese Patent Laid-Open (kokai) No. 51-113841). Then, the N-acyl-α-L-aspartyl-L-phenylalanine is separated from the mixture, and hydrogenated to obtain α-L-aspartyl-L-phenylalanine.

### Examples

Hereafter, the present invention will be still more specifically explained with reference to examples. Example 1: Deletion of *ydgT* gene in *Escherichia coli* W3110 strain
The *ydgT* gene on the chromosome of the *Escherichia coli* W3110 strain was deleted by the method (WO2005/010175) consisting of the combination of the method of Datsenko et al. (Datsenko, K.A. et al., Proc. Natl. Acad. Sci. USA, 97:6640-6645 (2000)) and an excision system derived from λ phage (Cho E.H. et al., J. Bacteriol., 184:5200-5203 (2002)) .

Specifically, PCR was performed by using the plasmid pMW118-attL-cat-attR (WO2005/010175) as a template DNA and synthetic oligo-DNAs shown in SEQ ID NOS: 1 and 2. pMW118-attL-cat-attR is a plasmid obtained by inserting the attL and attR genes, which are the attachment sites of λ phage, and the cat gene as an antibiotic resistance gene into pMW118 (Takara Bio), and they are inserted in the order of attL-cat-attR.

After PCR, the template DNA was fully digested with the restriction enzyme DpnI, and the obtained PCR product was purified with Wizard(R) SV Gel and PCR Clean-Up System (Promega, USA). This PCR product was introduced into the *Escherichia coli* W3110 strain containing the plasmid pKD46 having temperature sensitive replication ability (WO2005/010175, Datsenko, K.A. et al., Proc. Natl. Acad. Sci. USA., 97:6640-6645 (2000)) by electroporation. The plasmid pKD46 is a temperature sensitive plasmid containing the DNA fragment of 2154 nucleotides in total of λ phage (GenBank/EMBL accession number J02459, 31088th to 33241st nucleotides) containing the genes coding for the Red recombinase of λRed homologous recombination system (Υ, β and exo genes) controlled by the arabinose-inducible ParaB promoter. In order to prepare competent cells for electroporation, W3110/pKD46 was cultured at 30°C for 18 hours in 1 mL of the LB medium containing 100 mg/L of ampicillin. The obtained culture broth (300 µl) was added to the LB medium (50 mL) containing 100 mg/L of ampicillin and 1 mM L-arabinose, and flask culture was performed at 30°C for 3 hours. The obtained cells were washed 3 times with 10% glycerol to prepare competent cells for electroporation. The PCR product (about 100 ng) was added to 300 µL of the competent cells, and electroporation was performed. The cells subjected to electroporation were cultured at 37°C for 1 hour in 1 mL of the LB medium, and then inoculated on the LB agar medium (containing 40 mg/L of chloramphenicol). The cells were cultured at 37°C for 18 hours to obtain a chloramphenicol resistant strain, and thereby obtain a W3110 strain in which the *ydgT* gene on the chromosome was replaced with the attL-cat-attR gene. This strain was designated W3110ydgT::cat strain.

Then, in order to remove the attL-cat-attR gene from the W3110ydgT::cat strain, the helper plasmid pMW-intxis-ts (WO2005/010175) was used. pMW-intxis-ts is a plasmid carrying a gene coding for λ phage integrase (Int) and a gene coding for excisionase (Xis), and having temperature sensitive replication ability. If pMW-intxis-ts is introduced into a cell, recombination occurs between the plasmid and attL or attR on the chromosome, thus the gene between attL and-attR is excised, and only the attL or attR sequence remains on the chromosome.

Competent cells of the W3110ydgT::cat strain were prepared in a conventional manner, transformed with the helper plasmid pMW-intxis-ts, and cultured at 30°C for 24 hours on the LB agar medium containing 100 mg/L of ampicillin to select an ampicillin-resistant strain. Then, to remove the pMW-intxis-ts plasmid, the ampicillin-resistant transformant was inoculated on the LB agar medium and cultured at 37°C for 18 hours. Ampicillin resistance and chloramphenicol resistance of the obtained colonies were examined to obtain a *ydgT* gene-disrupted strain in which attL-cat-attR and pMW-intxis-ts had been eliminated, W3110ΔydgT. Disruption of the *ydgT* gene on the chromosome was confirmed by PCR using the synthetic DNAs shown in SEQ ID NOS: 7 and 8 as primers. Then, in order to impart an antibiotic resistance marker to the W3110ΔydgT strain, competent cells of W3110ΔydgT were produced in a conventional manner, and pSTV29 was introduced.

### Example 2: L-Lysine production with Escherichia coli L-lysine-producing bacterium deficient in ydgT gene (1)

L-Lysine production was performed by using *Escherichia coli* deficient in the *ydgT* gene. As the L-lysine-producing bacterium, the WC196ΔcadAΔldcC strain (also called "WC196LC strain", WO96/17930, European Patent No. 0796912) was used.

The *ydgT* gene on the chromosome of the WC196ΔcadAΔldcC strain was disrupted by P1 transduction using the W3110ydgT::cat strain, specifically, as follows. The W3110ydgT::cat strain was cultured at 37°C for 18 hours in 1 mL of the LB medium. The obtained culture broth (50 µL) was added to the LB medium (4 mL), and culture was performed at 37°C for 1 hour. The cells were collected from the culture broth, and suspended in fresh LB medium (1 mL, containing 5 mM calcium chloride). P1 phage was added to the cell suspension, and the suspension was left at 37°C for 1 hour. To the cell suspension infected with the P1 phage, 2.5 mL of R-Top Agar (containing 10 g/L of tryptone, 1 g/L of yeast extract, 8 g/L of NaCl, 8 g/L of agar, 2 mM CaCl₂, and 0.1% glucose) was added, and the mixture was overlaid on the LB agar medium, and incubated at 37°C for 14 hours. Then, 2 mL of the LB medium was added on the plate, the P1 phage and the cells were collected together with R-Top Agar, 30 µL of chloroform was added to them, and the supernatant was collected by centrifugation. After the addition of chloroform and collection of supernatant were repeated 3 times, the supernatant was filtered with a filter having a pore size of 0.2 µm to obtain a P1 phage solution for *ydgT* gene disruption.

Then, the WC196ΔcadAΔldcC strain was cultured at 37°C for 18 hours in 1 mL of the LB medium. The obtained culture broth (50 µl) was added to 4 mL of the LB medium, and culture was performed at 37°C for 1 hour. The cells were collected from the obtained culture broth, and suspended in fresh LB medium (1 mL, containing 5 mM calcium chloride), the P1 phage solution for *ydgT* gene disruption was added to the suspension, and the mixture was left at room temperature for 15 minutes. To the suspension, 5 mL of the LB medium containing 1% citric acid was added, the mixture was left at 30°C for 15 minutes, and then the cells were collected by centrifugation. The obtained cells were suspended in 5 mL of the fresh LB medium containing 1% citric acid, and the suspension was left at 30°C for 30 minutes. Then, the cells were washed twice with the LB medium, inoculated on the LB agar medium (containing 40 mg/L of chloramphenicol), and cultured at 37°C for 18 hours. In such a manner as described above, WC196ΔcadAΔ1dcC ydgT::cat strain was obtained, in which the *ydgT* gene was replaced with the attL-cat-attR gene.

Competent cells of the WC196ΔcadAΔ1dcC ydgT::cat strain obtained above were prepared in a conventional manner, transformed with the helper plasmid pMW-intxis-ts (WO2005/010175), and cultured at 30°C for 24 hours on the LB agar medium containing 100 mg/L of ampicillin to select an ampicillin-resistant strain. Then, to remove the pMW-intxis-ts plasmid, the ampicillin-resistant strain was inoculated on the LB agar medium and cultured at 37°C for 18 hours. Ampicillin resistance and chloramphenicol resistance of the obtained colonies were examined to obtain a *ydgT* gene-disrupted strain in which attL-cat-attR and pMW-intxis-ts had been eliminated, WC196ΔcadAΔ1dcCΔydgT. Disruption of the *ydgT* gene on the chromosome was confirmed by PCR using the synthetic DNAs shown in SEQ ID NOS: 7 and 8 as primers.

Into the obtained WC196ΔcadAΔ1dcCΔydgT strain and the parent strain thereof, WC196ΔcadAΔ1dcC strain, pCABD2 (WO95/16042) was introduced to obtain WC196ΔcadAΔ1dcCΔydgT/pCABD2 strain and WC196ΔcadAΔ1dcC/pCABD2 strain. Each of these two strains was inoculated on the LB agar medium (containing 100 mg/L of streptomycin), and cultured at 37°C for 24 hours. The grown cells were scraped up, and suspended in L-lysine production medium A [60 g/L of glycerol, 16 g/L of (NH₄)₂SO₄, 1.0 g/L of KH₂PO₄, 1.0 g/L of MgSO₄·7H₂O, 0.01 g/L of FeSO₄·7H₂O, 0.01 g/L of MnSO₄·7H₂O, 2.0 g/L of yeast extract, and 30 g/L of CaCO₃ (Wako Pure Chemical Industries)] containing 100 mg/L of streptomycin.

The obtained cell suspension was inoculated into 20 mL of the medium A at a specific turbidity OD660 of 0.1, and culture was performed at 37°C. The culture broth was repeatedly sampled in a volume of 0.5 mL during the culture, and specific turbidity of the culture broth (OD660), L-lysine concentration in the culture broth, and glycerol concentration in the culture broth were measured for each sample. In order to measure OD660, the culture broth was diluted 51 times with 0.1 M HCl, and specific turbidity at 660 nm was measured with a spectrophotometer DU-800 (Beckman Coulter, USA). The L-lysine concentration in the culture broth was measured for the culture broth diluted 20 times with water by using Biotech Analyzer AS310 (Sakura Seiki, Japan). The glycerol concentration in the culture broth was measured for the culture broth diluted 20 times or 40 times with water by using a multifunctional biosensor BF-5 (Oji Scientific Instruments). At each culture time, the measurement was performed for four samples, except that the measurement was performed for nine samples immediately after the start of the culture and at 48 hours after the start of the culture.

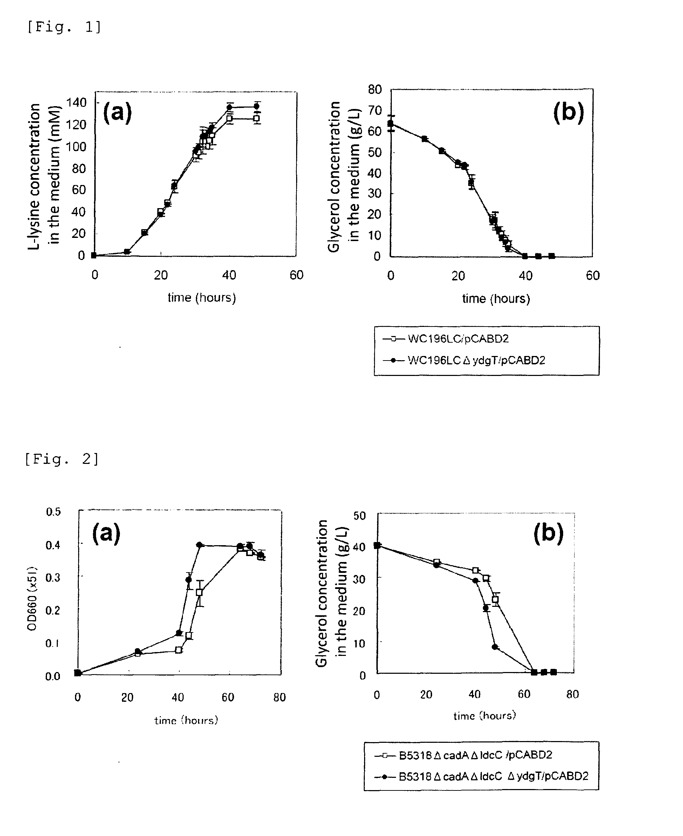

As a result, the L-lysine accumulation in the culture broth increased from 125.3 ± 5.0 mM to 136.3 ± 4.5 mM due to the deletion of the *ydgT* gene (Fig. 1). Furthermore, L-lysine yield based on glycerol was improved from 34.9 ± 1.5 (%) to 37.5 ± 1.5 (%).

### Example 3: L-Lysine production with Escherichia coli L-lysine-producing bacterium deficient in ydgT gene (2)

As the L-lysine-producing bacterium, the *Escherichia coli* VKPM B-5318 strain (European Patent No. 0593792, henceforth referred to as "B5318 strain"), in which the *cadA* gene and the *1dcC* gene were disrupted, was used.

The *cadA* gene and the *1dcC* gene coding for lysine decarboxylase on the chromosome of the B5318 strain were disrupted in the same manner as that used for disruption of the *ydgT* gene in Example 1.

Specifically, disruption was performed as follows. PCR was performed by using the plasmid pMW118-attL-cat-attR as a template DNA, and synthetic oligo-DNAs shown in SEQ ID NOS: 3 and 4 or SEQ ID NOS: 5 and 6, respectively. After PCR, the template DNA was fully digested with the restriction enzyme *Dpn*I*,* and the obtained PCR product was purified with Wizard(R) SV Gel and PCR Clean-Up System (Promega). Each PCR product was introduced into the *Escherichia coli* W3110 strain containing the plasmid pKD46 having temperature sensitive replication ability by electroporation. Competent cells for the electroporation were prepared in the same manner as that of Example 1. The PCR product (about 100 ng) was added to 300 µL of the competent cells, and electroporation was performed. The cells subjected to electroporation were cultured at 37°C for 1 hour in 1 mL of the LB medium, and then inoculated on the LB agar medium (containing 40 mg/L of chloramphenicol). The cells were cultured at 37°C for 18 hours to obtain a chloramphenicol resistant strain, and thereby obtain a W3110 strain in which the *cadA* gene or the *1dcC* gene on the chromosome was replaced with the attL-cat-attR gene.

Then, in order to successively disrupt the *cadA* gene and *1dcC* gene on the chromosome of the B5318 strain by P1 transduction using each of those gene-disrupted strains, P1 phages in which the chromosome of each disrupted strain was incorporated were prepared. First, the W3110 strain in which the *cadA* gene was disrupted was cultured at 37°C for 18 hours in 1 mL of the LB medium. The obtained culture broth (50 µl) was added to the LB medium (4 mL), and culture was performed at 37°C for 1 hour. The cells were collected from the obtained culture broth, and suspended in fresh LB medium (1 mL, containing 5 mM calcium chloride), P1 phage was added to the cell suspension, and the suspension was left at 37°C for 1 hour. R-Top Agar (2.5 mL) was overlaid on the cell suspension infected with the P1 phage, the cell suspension was inoculated on the LB agar medium, and culture was performed at 37°C for 14 hours. Then, 2 mL of the LB medium was added on the plate, the P1 phage and the cells were collected together with R-Top Agar, 30 µL of chloroform was added to them, and the supernatant was collected by centrifugation. After the addition of chloroform and collection of supernatant were repeated 3 times, the supernatant was filtered with a filter having a pore size of 0.2 µm to obtain a P1 phage solution for *cadA* gene disruption. Furthermore, a P1 phage solution for *1dcC* gene disruption was obtained in the same manner by using the W3110 strain in which the *1dcC* gene was disrupted.

Then, the *cadA* gene on the chromosome of the B5318 strain was disrupted by using the P1 phage for *cadA* gene disruption, specifically, as follows. The B5318 strain was cultured at 37°C for 18 hours in 1 mL of the LB medium. The obtained culture broth (50 µL) was added to the LB medium (4 mL), and culture was performed at 37°C for 1 hour. The obtained cells were collected, and suspended in fresh LB medium (1 mL, containing 5 mM calcium chloride), P1 phage was added to the cell suspension, and the suspension was left at room temperature for 15 minutes. To the suspension, 5 mL of the LB medium containing 1% citric acid was added, the mixture was left at 30°C for 15 minutes, and then the cells were collected by centrifugation. The obtained cells were suspended in 5 mL of fresh LB medium containing 1% citric acid, and the suspension was left at 30°C for 30 minutes. Then, the cells were washed twice with the LB medium, inoculated on the LB agar medium (containing 40 mg/L of chloramphenicol), and cultured at 37°C for 18 hours to obtain B5318 cadA::cat strain, in which the *cadA* gene was replaced with the attL-cat-attR gene.

Competent cells of the B5318 cadA::cat strain obtained above were prepared in a conventional manner, transformed with the helper plasmid pMW-intxis-ts, and cultured at 30°C for 24 hours on the LB agar medium containing 100 mg/L of ampicillin to select an ampicillin-resistant strain. Then, to remove the pMW-intxis-ts plasmid, the ampicillin-resistant strain was inoculated on the LB agar medium and cultured at 37°C for 18 hours. Ampicillin resistance and chloramphenicol resistance of the obtained colonies were examined to obtain a *cadA* gene-disrupted strain, B5318ΔcadA strain, in which attL-cat-attR and pMW-intxis-ts had been eliminated. Disruption of the *cadA* gene on the chromosome was confirmed by PCR using the synthetic DNAs shown in SEQ ID NOS: 9 and 10 as primers.

Then, by using the P1 phage for *1dcC* gene disruption, the *1dcC* gene of the B5318ΔcadA strain was disrupted, and attL-cat-attR and pMW-intxis-ts were further eliminated in the same manner as that described above to obtain B5318ΔcadAΔ1dcC strain. From this strain, by using the P1 phage for *ydgT* gene disruption, the *ydgT* gene of this strain was disrupted, and attL-cat-attR and pMW-intxis-ts were further eliminated to obtain B5318ΔcadAΔ1dcCΔydgT strain. Disruption of the *1dcC* gene on the chromosome was confirmed by PCR using the synthetic DNAs shown in SEQ ID NOS: 11 and 12 as primers. Furthermore, disruption of the *ydgT* gene on the chromosome was confirmed by PCR using the synthetic DNAs shown in SEQ ID NOS: 8 and 9 as primers.

pCABD2 was introduced into the B5318ΔcadAΔ1dcCΔydgT strain and the B5318ΔcadAΔ1dcC strain to obtain B5318ΔcadAΔ1dcCΔydgT/pCABD2 strain and B5318ΔcadAΔ1dcC/pCABD2 strain.

Each of these two strains was inoculated on the LB agar medium (containing 100 mg/L of streptomycin), and cultured at 37°C for 24 hours. The grown cells were scraped up, and suspended in L-lysine production medium B [40 g/L of glycerol, 16 g/L of (NH₄)₂SO₄, 1.0 g/L of KH₂PO₄, 1.0 g/L of MgSO₄·7H₂O, 0.01 g/L of FeSO₄·7H₂O, 0.01 g/L of MnSO₄·7H₂O, 2.0 g/L of yeast extract, and 30 g/L of CaCO₃ (Wako Pure Chemical Industries)] containing 100 mg/L of streptomycin. The obtained cell suspension was inoculated into 20 mL of the medium B at a specific turbidity OD660 of 0.1, and culture was performed at 37°C. Culture was performed with three samples for each strain, and specific turbidity of the culture broth (OD660), L-lysine concentration in the culture broth, and glycerol concentration in the culture broth were measured for each sample.

As a result, improvement in the proliferation rate and glycerol-assimilating ability due to the disruption of the *ydgT* gene was observed (Fig. 2).

## Claims

1. A method for producing an L-amino acid comprising culturing a bacterium belonging to *γ-Proteobacteria* and having an ability to produce the L-amino acid in a medium containing glycerol as a carbon source to produce and accumulate the L-amino acid in the medium, and collecting the L-amino acid from the medium, wherein the bacterium is a bacterium modified so that the activity of the Cnu protein is reduced.

2. The method according to claim 1, wherein the activity of the Cnu protein is reduced by disrupting the *ydgT* gene coding for the Cnu protein on the chromosome, or by reducing expression amount of the gene.

3. The method according to claim 1 or 2, wherein the Cnu protein is a protein defined in the following (A) or (B):
(A) a protein having the amino acid sequence shown in SEQ ID NO: 14,
(B) a protein having the amino acid sequence shown in SEQ ID NO: 14, but including substitution, deletion, insertion or addition of one or several amino acid residues, reduction of which activity in the bacterium results in improvement in the ability to produce the L-amino acid.

4. The method according to claim 2 or 3, wherein the *ydgT* gene is a DNA defined in the following (a) or (b):
(a) a DNA comprising the nucleotide sequence of SEQ ID NO: 13,
(b) a DNA hybridizable with a nucleotide sequence complementary to the nucleotide sequence of SEQ ID NO: 13 or a probe that can be prepared from the nucleotide sequence under stringent conditions, and coding for a protein, reduction of which activity in the bacterium results in improvement in the ability to produce the L-amino acid.

5. The method according to any one of claims 1 to 4, wherein the L-amino acid is an aspartic acid type amino acid.

6. The method according to claim 5, wherein the L-amino acid is L-lysine.

7. The method according to any one of claims 1 to 6, wherein the bacterium is a bacterium belonging to the family *Enterobacteriaceae.*

8. The method according to claim 7, wherein the bacterium is an *Escherichia* bacterium.

9. The method according to claim 8, wherein the bacterium is *Escherichia coli.*


SEQUENCE LISTING

<110>  Ajinomoto Co., Inc.

<120>  A method for producing an L-amino acid

<130>  D190-10201

<150>  JP2009-255042
<151>  2009-11-06

<160>  14

<170>  PatentIn version 3.5

<210>  1
<211>  70
<212>  DNA
<213>  Artificial

<220>
<223>  primer

<400>  1
atacctaccc ctttggtatt aattatttta tggacctttt tgaagcctgc ttttttatac      60
taagttggca                                                              70

<210>  2
<211>  70
<212>  DNA
<213>  Artificial

<220>
<223> primer

<400> 2
ggcgttatta taaggctttt ataaccgatc gctactttct cgctcaagtt agtataaaaa 60
agctgaacga 70

<210> 3
<211> 70
<212> DNA
<213> Artificial

<220>
<223> primer

<400> 3
   catgtgttgg gaggggcctt ttttacctgg agatatgact tgaagcctgc ttttttatac 60
   taagttggca 70

<210> 4
<211> 70
<212> DNA
<213> Artificial

<220>
<223> primer

<400> 4
aaaaagggaa gtggcaagcc acttcccttg tacgagctaa cgctcaagtt agtataaaaa 60
agctgaacga 70

<210> 5
<211> 70
<212> DNA
<213> Artificial

<220>
<223> primer

<400> 5
agcaggctat gattaaggaa ggattttcca ggaggaacac tgaagcctgc ttttttatac 60
taagttggca 70

<210> 6
<211> 70
<212> DNA
<213> Artificial

<220>
<223> primer

<400> 6
gttaacagca cgttactcgc ccggaagccg ctctggcaag cgctcaagtt agtataaaaa 60
agctgaacga 70

<210> 7
<211> 20
<212> DNA
<213> Artificial

<220>
<223> primer

<400> 7
ttgtaataac acaagcgttc 20

<210> 8
<211> 19
<212> DNA
<213> Artificial

<220>
<223> primer

<400> 8
ttgtggtggt cataggttc 19

<210> 9
<211> 20
<212> DNA
<213> Artificial

<220>
<223> primer

<400> 9
accgcgtcta acgcacatta 20

<210> 10
<211> 20

<212>  DNA
<213>  Artificial

<220>
<223>  primer

<400>  10
gagggtgttt tcatgtgttc                                                    20

<210>  11
<211>  20
<212>  DNA
<213>  Artificial

<220>
<223>  primer

<400>  11
gcaggctatg attaaggaag                                                    20

<210>  12
<211>  20
<212>  DNA
<213>  Artificial

<220>
<223>  primer

<400>  12
atcgcaatat ggtgaacctg                                                    20

<210> 13

<211> 216

<212> DNA

<213> Escherichia coli

<220>

<221> CDS

<222> (1)..(216)

<400> 13

atg act gtt cag gac tac tta tta aaa ttt cgc aaa atc agt tca ctc 48

Met Thr Val Gln Asp Tyr Leu Leu Lys Phe Arg Lys Ile Ser Ser Leu

1               5                   10                  15

gaa agt ctg gaa aaa ctc tac gac cat ctt aat tac acc ctg acg gac 96

Glu Ser Leu Glu Lys Leu Tyr Asp His Leu Asn Tyr Thr Leu Thr Asp

            20                  25                  30

gat cag gaa ctg atc aat atg tat cgt gct gcc gat cac cgt cgc gca 144

Asp Gln Glu Leu Ile Asn Met Tyr Arg Ala Ala Asp His Arg Arg Ala

        35                  40                  45

gag ctg gtt tct ggc ggg cgt ttg ttt gac ctc ggc cag gta ccg aag 192

Glu Leu Val Ser Gly Gly Arg Leu Phe Asp Leu Gly Gln Val Pro Lys

    50                  55                  60

tcc gtc tgg cac tat gtc caa taa 216

Ser Val Trp His Tyr Val Gln

65                  70

<210> 14

<211> 71

<212> PRT

<213> Escherichia coli

<400> 14
Met Thr Val Gln Asp Tyr Leu Leu Lys Phe Arg Lys Ile Ser Ser Leu
1               5                   10                  15
Glu Ser Leu Glu Lys Leu Tyr Asp His Leu Asn Tyr Thr Leu Thr Asp
            20                  25                  30
Asp Gln Glu Leu Ile Asn Met Tyr Arg Ala Ala Asp His Arg Arg Ala
        35                  40                  45
Glu Leu Val Ser Gly Gly Arg Leu Phe Asp Leu Gly Gln Val Pro Lys
    50                  55                  60
Ser Val Trp His Tyr Val Gln
65                  70